# EUROPEAN PATENT APPLICATION

(11) **EP 1 211 275 A1**
(43) Date of publication of application: **05.06.2002**
(21) Application number: 00126193.2
(22) Date of filing: 30.11.2000
(51) Int. Cl.: C08G 69/14, C08G 69/16, C08G 69/22, C07D 223/10, C07D 201/04

(54) **Modified Polylactam**

(71) Applicant: DSM N.V., 6411 TE Heerlen (NL)
(72) Inventor: Nijenhuis, Atza Jan, 6132 HB Sittard (NL)

(57) **Abstract**

The invention relates to a modified polylactam containing recurring units of lactam and minor amounts of a lactam derivative having one or more non-polymerisable functional groups directly or indirectly attached to the carbon atoms of the lactam ring and wherein the functional group comprises a heteroatom. The invention further relates to a process for the preparation of the polylactam, to the use of the modified polylactam, to fibres made from the modified lactam and to the lactam derivative and its production.

## Description

The invention relates to a modified polylactam containing recurring units of lactam and minor amounts of a lactam derivative. The invention further relates to a process for the preparation of the modified polylactam, to the use of the modified polylactam, to fibres made from the modified polylactam and to the lactam derivative and its production.

Polyamide-6 can be made suitable for differential dyeing by introducing sulfonate groups in the polymer. This makes it possible to dye the fibre using both cationic and anionic dyes. These fibres also have anti-stain properties, which means that it is difficult for acid-based impurities to attach themselves to these fibres. Such polyamides-6 are mainly used for the production of carpet yarns, but also for fibres, paper coatings and glues.

US 5,164,261 describes shaped articles, such as fibres, formed of a polyamide formed by salt-blending the polyamide precursor salt with a cationic dye modifier, such as the dimethyl ester of 5-sulfoisophthalic acid, followed by polymerisation. The articles are rendered resistant to staining by acid dyes at ambient conditions either by adding an acid dye to the polymer melt or by dyeing with an acid dye from an acid dyebath at pH 2 to 7 and 60°C to 100°C. so that the shaped article contains from at least 0.0048 and preferably at least 0.0096 wt % of an acid dyestuff. In a preferred aspect of the invention, the shaped articles are oriented, crimped, heat-set fibres for use as the facing in carpets.

From the state of the art of US 3,950,311 it is known to introduce a sulfonate group by using an alkali metal salt of 5-sulfoisophthalic acid in combination with hexamethylenediamine (hereinafter referred as Li-sip system). For this purpose, the lithium or sodium salts can be used. These compounds are added during the polymerisation of polyamide-6 from caprolactam. The polymer is used to form products such as fibres or foils.

US 4,579,762 describes a similar process only using an alkali metal salt of 5-sulfoisophthalic acid without hexamethylenediamine.

However, the use of these compounds greatly affects the lactam polymerisation and results in several complications during the polymerisation. The reason for that is, that the carboxy groups and the amino groups of the additives interfere with the lactam and the polyamide chains. So the compounds act as bifunctional chainstoppers and influence the molecular weight of the resulting polyamide. Furthermore, the solubility of the additives, the affects on the acidity and the balance between the amount of dicarboxylic acids and diamines are factors that complicate the polymerisation and often cause problems. In addition, the thermal properties of the polyamide are affected, the melting point is lowered and interference with the crystallisation of the polyamide occurs.

Therefore, it was the object of the present invention to prepare a modified polylactam with a new additive, which interferes with the lactam polymerisation to a lesser extent and adds functionality to the resulting modified polylactam.

This object of the invention is achieved by a modified polylactam containing recurring units of lactam and minor amounts of a lactam derivative having one or more non-polymerisable functional groups directly or indirectly attached to the carbon atoms of the lactam ring and wherein the functional group comprises a heteroatom.

The term "heteroatom" as used within the description covers all atoms except carbon atom and hydrogen atom. It is preferred that the heteroatom is selected from the group consisting of N-atom, O-atom, P-atom, S-atom, halogen-atom. Most preferred are N-atom, O-atom, P-atom, S-atom. The heteroatom containing functional groups can be further substituted with carbon or hydrogen containing groups, preferably alkyl-groups.

The term "functional group" as used within the description is defined as a group that actively takes part in stabilization, dyeability and flame retardancy of the resulting polymer.

In a preferred embodiment the lactam or the lactam derivative is selected from the group consisting of caprolactam, lauryllactam, caprylolactam or mixtures thereof. In a most preferred embodiment the lactam and the lactam derivative are both caprolactam, lauryllactam, caprylolactam, so that the resulting polylactam is made from caprolactam and modified caprolactam or from lauryllactam and modified lauryllactam or from caprylolactam and modified caprylolactam.

In a further preferred embodiment the lactam derivative contains a substituted or unsubstituted phenyl group between the functional group and the lactam group. Preferably the lactam derivative is substituted by a group according to formula I 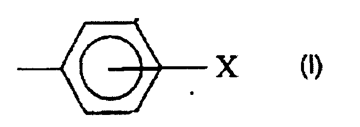 wherein X is a -SO₃H group, a ―SO₂NH₂ group, a OH group, a phosphate group, a NHR₁ group or a OR₂ group or salts thereof and R₁, R₂ independently are a C₁-C₄ alkyl group, a cycloalkyl group, or a phenyl group, and wherein the phenyl ring can be substituted at each position of the carbon atom. As salts monovalent, bivalent or trivalent salts can be used, preferably salts selected from the group consisting of Cu, Al, Mg, Zn, Ca, Sn, Fe salts or mixtures thereof. Most preferred are alkali metal salts, for example Li-salt, Na-salt, K-salt.

Further examples for groups substituting the lactam derivative are selected from t-butyl phenol, phenyl sulfonate, phenyl sulfonamide, phenyl phosphate, phosphate, phenol, and salts thereof.

In a most preferred embodiment the lactam derivative is substituted by a group of the formula II 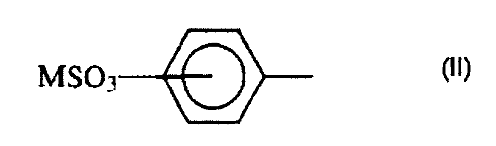 wherein one or more ―SO₃M groups are present at the phenyl ring and M is lithium, sodium or potassium, wherein the caprolactam derivative having the formula III, 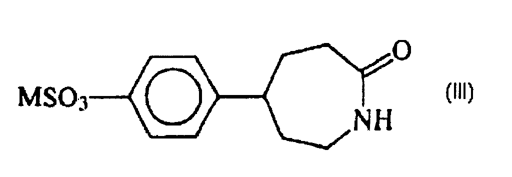 wherein M is lithium, sodium, or potassium, is mostly preferred.

As the functional group is not polymerisable the modified polylactam can contain up to 20 mol-%, preferably 0.001 to 20 mol-%, of the lactam derivative. These lactam derivative can be added during polymerisation without disturbing the polymerisation process and without a risk of gel formation.

The advantage of these compounds is, that they influence the polymerisation process to a lesser extent than the known systems of the state of the art. Thus the molecular weight and the molecular weight distribution is hardly changed. Furthermore, they do not interfere with the N-group balance and do not affect the acidity of the lactam melt.

The modified polylactam of the invention is prepared in the presence of the lactam derivative in the usual way. The polymerisation procedure incorporating the new lactam derivatives is exactly the same as for the normal lactam polymerisation. The resulting modified polylactam has essentially the same molecular weight as the normal polylactam. Furthermore, the thermal properties of the polymer are less affected in comparison with a polylactam obtained using the dicarboxylic acid/diamine system, for example with the lithium salt of 5-sulfoisophthalic acid and hexamethylenediamine.

The modified polylactam can be subjected to a postcondensation step to raise the M_{w}. Before the postcondensation step the modified polylactam has a M_{w} of 2000 to 35000, preferably 20000 to 30000 and most preferably 20000 to 25000. After postcondensation the modified polylactam has a M_{w} of 30000 to 60000, preferably 40000 to 60000 and most preferably 45000 to 55000.

The modified polylactam is used for fibres, paper coatings, glues, carpet yarns and engeneering plastics. Fibres made from the modified polylactam can further comprise usual polylactam. In this case the modified polylactam is incorporated in the polylactam by using a masterbatch of the modified polylactam, which is mixed with the regular polylactam.

The invention further relates to the new lactam derivative taking actively part in stabilisation, dyeability, and flame retardancy of the polylactam without affecting the molecular weight and the molecular weight distribution of the resulting polylactam. The most preferred lactam derivative is the caprolactam derivative having the formula III, 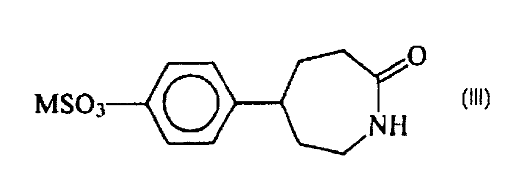 wherein M is lithium, sodium, or potassium.

The lactam derivative can be produced in the usual way by the following steps:
a. oximation reaction of a substituted cycloketone to the hydroxylimine of the ketone,
b. contacting with concentrated sulfuric acid to affect Beckmann rearrangement of hydroxylimine.

In the case the lactam derivative is the compound according to formula III a further advantage is that the compound can be obtained using the normal caprolactam synthesis starting from phenyl-cyclohexanone wherein the Beckmann rearrangement and the sulfonisation is made in one step according to the following procedure:
a. oximation reaction of phenyl cyclohexanone to the hydroxylimine of phenyl cyclohexanone,
b. contacting with concentrated sulfuric acid to affect Beckmann rearrangement of hydroxylimine of phenyl cyclohexanone and sulfonisation of phenyl ring in one step to give sulfonated phenyl caprolactam,
c. addition of alkalihydroxide and reaction of the sulfonated phenyl caprolactam to the alkali salt of sulfonated phenyl caprolactam.

Thus it is possible to produce the new additives in a usual caprolactam producing facility.

The new lactam derivatives according to the invention have a number of advantages over the existing systems of the state of the art using dicarboxylic acid salts and diamines. The lactam derivative does hardly interfere with the lactam polymerisation during the production of polylactam. As can be seen from the examples, a good incorporation of the lactam derivative takes place during the polylactam polymerisation. The use of the lactam derivative according to the invention has only a little effect on the molecular weight and molecular weight distribution compared to the known systems. The effect of the lactam derivative according to the invention on the thermal properties of the polymer is smaller than that of the known systems of the state of the art. Moreover, the use of the lactam derivative in the modified polylactam results in a higher melt viscosity at the same molecular weight in comparison with unmodified polylactam, which does not contain this additive.

A further finding is, that the lactam derivative containing polymers, have a higher zero shear melt viscosity. The increase of the zero shear melt viscosity is at least 5 %, preferably at least 10 % to 15 %, and most preferably at least 20 %. This is advantageous for blow moulding or film forming applications.

Therefore, the new lactam derivative according to the invention is a good alternative for the known additive systems containing dicarboxylic acid salts and diamines.

The invention will be further described on the basis of the following examples without being limited thereto.

### Examples

### Example 1

### Monomer synthesis of the lithium salt of 5-(1-(4-sulfonic acid) ―phenyl) ―hexahydro- 2H-azepine-2-one (5)

The starting material was 4-phenyl-cyclohexanone (**1**) and the process is described as follows:

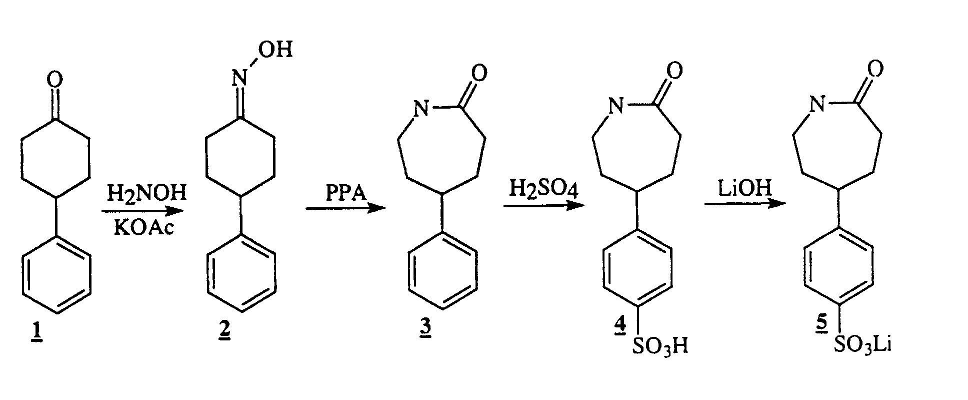

### Hydroxylimine of 4-phenyl-cyclohexanone (2)

4-phenyl-cyclohexanone (**1**) (103 g, 0.59 mole) was dissolved in methanol (1 L).
This mixture was cooled to 0°C, after which potassium acetate (173 g) and hydroxylammonium chloride (73 g) were added. The reaction mixture was then stirred for 16 hours upon which the temperature slowly rose to room temperature. Subsequently, the methanol was evaporated and the residue poured out into 1.5 L water and stirred for 10 min. The solid was removed by filtration, washed with water and dried. This resulted in 114 g of compound **2** (quantitative yield) being isolated as a white solid that was pure enough for the next step.

### 4-phenyl-hexahydro-2H-azepine-2-one (3)

The non-purified hydroxylimine **2** (108 g, max. 0.56 mole) was added in a short time to a mechanically stirred mixture of p-xylene (300 mL) and polyphosphoric acid (300 g). Subsequently, another 300 mL p-xylene was added and the mixture was heated for 3 hours until the boiling point was reached. Afterwards, the mixture was cooled to room temperature and 500 mL water was added with adequate stirring. This was followed by extraction with 1L chloroform. The organic layer was washed with water, dried (Na₂SO₄) and evaporated. The product was recrystallised from approx. 600 mL ethanol so that 83.1 g (0.44 mole, 75 %) (2 steps from **1**) of compound 3 could be isolated as a white solid.

### 5-(1-(4-sulfonic acid) ―phenyl)―hexahydro-2H-azepine-2-one (4)

Phenyl caprolactam **3** (54.2 g, 287 mmol) was added in portions to 150 ml 96% sulphuric acid. During the addition, the temperature of the mixture rose to 55°C. After everything had been added (in approximately 15 minutes) the mixture was stirred for 45 minutes at 60-65°C. Next, the reaction mixture was poured into 750 mL ice water and stirred for 30 minutes, the precipitate formed being filtered off and washed with methanol. After drying, the crude yield was 55.1 g. The filtrate was left to stand for a weekend, upon which precipitate was again formed, which was filtered off and dried, so that a second portion of 14.7 g could be isolated. The two portions were combined, brought to the boil in methanol for a short time and cooled to room temperature. The precipitate was filtered, washed twice with methanol and dried, so that 67 g (249 mmol, 87%) of pure sulfonic acid **4** could be isolated.

### Lithium salt of 5-(1-(4-sulfonic acid)-phenyl)-hexahydro-2H-azepine-2-one (5)

Sulfonic acid **4** (75,5 g, 281 mmol) was stirred in 400 ml water. Subsequently, LiOH H₂O (11.8g, 281 mmol) was added and the mixture was heated to 50°C so that a clear solution was formed. 200 mL water was evaporated and the residue was added to 200 mL acetone with vigorous stirring. The precipitate formed was filtered off, washed with acetone and dried so that 47.9 g (174 mmol, 62%) pure Li-salt **5** could be isolated. Expected elementary analysis: C 52.36%, H 5.13%, N 5.09%, Li 2.52%. Found: C 51.81%, H 5.06%, N 4.93%, Li 2.46%. (Purity on basis of Li and N is 97%)

### Example 1a

### Monomer synthesis of the lithium salt of 5-(1-(4-sulfonic acid) ―phenyl)―hexahydro- 2H- azepine-2-one (5) (alternative route)

The process for the preparation of compound (**5**) is made in the same way as in example 1 with the difference that compound (**2**) is contacted with concentrated sulfuric acid to affect Beckmann rearrangement of hydroxylimine of phenyl cyclohexanone and sulfonisation of phenyl ring in one step to give sulfonated phenyl caprolactam (**4**).

### Example 2

### Polymerisation of ε- caprolactam to modified polycaprolactam

To the mixture of caprolactam, aminocaproic acid and water 2.2 wt-% of the lithium salt of 5-(1-(4-sulfonic acid) ―phenyl) ―hexahydro- 2H- azepine-2-one (hereinafter referred as Li-salt) was added after which the normal polymerisation and treatment procedure for the production of polycaprolactam was carried out.

### Example 3

### Polymerisability of the lithium salt of 5-(1-(4-sulfonic acid)―phenyl)―hexahydro-2H- azepine-2-one

It was investigated whether the Li-salt was incorporated during the polymerisation in the modified polycaprolactam. This was done by determining the concentrations of sulphur by X-ray fluorescence in the washed polymer. Non-polymerised Li-salt is water-soluble and is therefore removed during the washing step. It was found that the amount of the Li-salt incorporated in the polymer was 90%.

**Table 1:**

| Incorporation during polymerisation of caprolactam with 2.2 wt-% Li-salt | | |
|---|---|---|
| | found | theory |
| Sulphur | 0.21 (± 0.02) wt-% | 0.22 wt-% |

Table 1 shows that the sulphur content found is very close to the theoretical value. On the basis of the sulphur content, Li-salt has the same reactivity relative to the polymerisation as caprolactam.

### Example 4

### Molecular weight and rheology of modified polycaprolactam versus blank polycaprolactam

The molecular weight and the rheology of the modified polycaprolactam was measured and compared with blank polycaprolactam.

**Table 2:**

| Influence of Li-salt on the molecular weight and the rheology | | | | | |
|---|---|---|---|---|---|
| | SEC-DV | | | | |
| | Mw/Mn | Mw (x10³) | Mz/Mw | [η]_{HCOOH} dl/g | η _{0 230C} Pas |
| **as-polymerised** | | | | | |
| | | | | | |
| blank polycaprolactam (comparison) | 2.2 | 25 | 1.6 | 1.05 | 5.9x10² |
| polycaprolactam with 0.08 meq/g Li-salt (invention) | 1.9 | 22 | 1.6 | 0.89 | 7.2x10² |
| | | | | | |

| **Post-condensed** | | | | | |
|---|---|---|---|---|---|
| | | | | | |
| blank polycaprolactam (comparison) | 2.2 | 52 | 1.9 | 1.68 | 1.3x10⁴ |
| polycaprolactam with 0.08 meq/g Li-salt (invention) | 2.0 | 49 | 1.8 | 1.57 | 1.4x10⁴ |
| SEC-DV = size exclusion chromatography-differential viscosimetry | | | | | |
| Mw/Mn = average molecular weight/number average molecular weight | | | | | |
| Mw = average molecular weight | | | | | |
| Mz/Mw = viscosity average molecular weight/ average molecular weight | | | | | |
| [η]_{HCOOH} = intrinsic viscosity measured in formic acid | | | | | |
| η _{0 230C} = zero shear melt viscosity | | | | | |
| Pas = Pascalseconds | | | | | |
| meq = milliequivalents | | | | | |

From Table 2 it is concluded that the addition of Li-salt has only very little effect on the molecular weight and the molecular weight distribution of the polymer. The SEC-DV results indicate that there is a small decrease in the molecular weight. It is evident from the fact that the Li-salt does not function as a chain stopper (causes non-reactive chain ends) that the polymer can be subjected to post-condensation up to a very high molecular weight, just like the blank polycaprolactam. It should be noted that after post-condensation the differences in molecular weight are within the reproducibility of the polymerisation experiments.

A further finding is, that the Li-salt containing polymers have a higher zero shear melt viscosity. The increase of the zero shear melt viscosity in the present example is about 18 %. This is advantageous for blow moulding or film forming applications.

### Example 5

### Thermal properties of modified polycaprolactam versus blank polycaprolactam

The thermal properties of the modified polycaprolactam of the invention were measured and compared with blank polycaprolactam. Table 3 shows the results.

**Table 3:**

| Influence of comonomers on thermal properties | | | | | |
|---|---|---|---|---|---|
| | Tm₂ (°C) | ΔHₕ₂ (J/g) | Tcc (°C) | ΔHc₁ (J/g) | Tg (°C) |
| **as-polymerised** | | | | | |
| | | | | | |
| blank polycaprolactam (comparison) | 218.8 | 77.4 | 171.1 | 73.5 | 44 |
| polycaprolactam with 0.08 meq/g Li-salt (invention) | 216.0 | 65.7 | 164.6 | 66.2 | 45 |
| polycaprolactam with 0.08 meq/g Li-sip + 0.06 meq/g HMDA (comparison) | 212.8 | 64.2 | 160.4 | 62.1 | 63 |
| | | | | | |

| **post-condensed** | | | | | |
|---|---|---|---|---|---|
| | | | | | |
| blank polycaprolactam (comparison) | 220.2 | 76.3 | 167.7 | 76.3 | 56 |
| polycaprolactam with 0.08 meq/g Li-salt (invention) | 215.8 | 74.3 | 161.9 | 71.3 | 48 |

As evident from Table 3, the incorporation of Li-salt reduces the melting temperature (Tm₂), the crystallisation temperature during cooling (Tcc), and the melting heat formed during both melting and cooling (ΔHₕ₂, ΔHc₁). Still, there is a clear improvement in these properties compared to the known system (lithium salt of 5-sulfoisophthalic acid/Hexamethylenediamine hereinafter referred as Li-sip system) as currently in commercial use. Compared to blank polycaprolactams, post-condensation increases the difference in Tm₂, Tcc and Tg, whereas the difference in melting and crystallisation heat decreases.

## Claims

1. Modified polylactam containing recurring units of lactam and minor amounts of a lactam derivative having one or more non-polymerisable functional groups directly or indirectly attached to the carbon atoms of the lactam ring and wherein the functional group comprises a heteroatom.

2. Modified polylactam according to claim 1, **characterised in that** the lactam or the lactam derivative is selected from the group consisting of caprolactam, lauryllactam, caprylolactam or mixtures thereof.

3. Modified polylactam according to claim 1 or 2, **characterised in that** the lactam derivative contains a substituted or unsubstituted phenyl group between the functional group and the lactam group.

4. Modified polylactam according to claims 1 to 3, **characterised in that** the lactam derivative is substituted by a group according to formula I 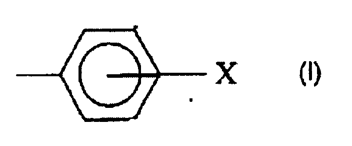 wherein X is a -SO₃H group, a ―SO₂NH₂ group, a OH group, a phosphate group, a NHR₁ group or a OR₂ group and salts thereof and R₁, R₂ independently are a C₁-C₄ alkyl group, a cycloalkyl group, or a phenyl group, and wherein the phenyl ring can be substituted at each position of the carbon atom.

5. Modified polylactam according to claims 1 to 4 ,**characterised in that** the lactam derivative is substituted by groups selected from t-butyl phenol, phenyl sulfonate, phenyl sulfonamide, phenyl phosphate, phosphate, phenol, and salts thereof.

6. Modified polylactam according to claims 1 to 5, **characterised in that** the lactam derivative is substituted by a group of the formula II 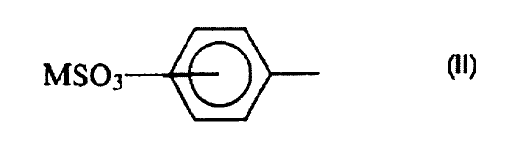 wherein one or more ―SO₃M groups are present at the phenyl ring and M is lithium, sodium or potassium.

7. Modified polylactam according to claims 1 to 6, **characterised in that** the lactam derivative is a caprolactam derivative having the formula III, 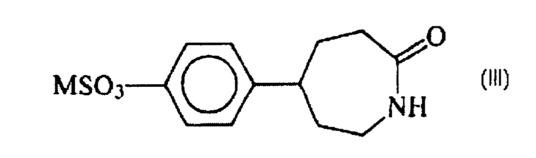 wherein M is lithium, sodium, or potassium.

8. Modified polylactam according to claims 1 to 7, **characterised in that** it contains 0.001 to 20 mol-% of the lactam derivative.

9. Process for the preparation of a modified polylactam according to claims 1 to 8, **characterised in that** the lactam polymerisation is carried out in the presence of a functionalised lactam derivative as described in claims 1 to 8.

10. Process according to claim 9, **characterised in that** the modified polylactam is subjected to a postcondensation.

11. Use of the modified polylactam according to claims 1 to 8 for fibres, paper coatings, glues, carpet yarns, engeneering plastics.

12. Fibres comprising the modified polylactam according to claims 1 to 8.

13. Fibres according to claim 11 further comprising polylactam.

14. Lactam derivative that takes actively part in stabilisation, dyeability, and flame retardancy of the polylactam without affecting the molecular weight and the molecular weight distribution of the resulting polylactam.

15. Lactam derivative as described in claims 1 to 8.

16. Use of the lactam derivative according to claims 13 or 14 for adding functionality to polylactam without affecting the molecular weight and the molecular weight distribution of the resulting polylactam.

17. Process for the manufacture of the lactam derivative according to claims 1 to 7 by the following steps:
a. oximation reaction of a substituted cycloketone to the hydroxylimine of the ketone,
b. contacting with concentrated sulfuric acid to affect Beckmann rearrangement of hydroxylimine.

18. Process for the preparation of the lactam derivative according to claim 6 or 7, **characterised by** the following steps:
a. oximation reaction of phenyl cyclohexanone to the hydroxylimine of phenyl cyclohexanone,
b. contacting with concentrated sulfuric acid to affect Beckmann rearrangement of hydroxylimine of phenyl cyclohexanone and sulfonisation of phenyl ring in one step to give sulfonated phenyl caprolactam,
c. addition of alkalihydroxide and reaction of the sulfonated phenyl caprolactam to the alkali salt of sulfonated phenyl caprolactam.
